# EUROPEAN PATENT APPLICATION

(11) **EP 1 495 704 A1**
(43) Date of publication of application: **12.01.2005**
(21) Application number: 03077161.2
(22) Date of filing: 10.07.2003
(51) Int. Cl.: A47K 7/03, A61F 13/15

(54) **Fibrous web product**

(71) Applicant: SCA Hygiene Products AB, 405 03 Göteborg (SE)
(72) Inventor: Müller, Heinz Jürgen, 68 623 Lampertheim (DE)
(74) Representative: Egeröd, Lisbeth

(57) **Abstract**

A fibrous web product comprising at least two outer plies (13,14) of fibrous web material and an inner material layer (15) interposed between the outer plies, said inner material layer comprising a bulky soft material. A barrier layer (16) of an at least substantially liquid impervious material is applied between at least one of said outer plies (13,14) and the inner material layer (15) and at least one microencapsulated functional substance (17) is applied between the barrier layer (16) and said outer ply (13).

## Description

### TECHNICAL FIELD

The present invention refers to a fibrous web product with integrated hygiene, cleaning, disinfectant, medical treatment or other function, said product comprising at least two outer plies of fibrous web material and an inner material layer interposed between the outer plies, said inner material layer comprising a bulky soft material.

### BACKGROUND OF THE INVENTION

Fibrous web products, such as tissue paper products and nonwoven products are daily used for personal hygiene, in kitchens, in healthcare, in industries, in workshops etc. Examples of tissue products are toilet tissue, kitchen tissue, facial tissue, paper towels, paper napkins, industrial wiping material etc. It is very common to laminate two or more tissue plies in order to produce the final tissue product. The absorbent capacity and the bulk of the tissue product are herewith improved. The tissue plies are laminated together by embossing or gluing or a combination of both. Various types of nonwoven materials may also be used for the above purposes.

For certain applications, such as cosmetic pads, napkins, high bulk wipes etc. it is desired to provide tissue products having an increased bulk, absorbency and barrier function against the passage of fluid, than can be achieved by laminating two or more tissue plies together.

GB-A-2 281 212 discloses a cosmetic pad comprising a layer of non-absorbent material, such as a thermoplastic film material, which is bonded to an absorbent fibrous web material containing a certain proportion of thermoplastic fibers. The two layers are secured together by ultrasonic bonding. The pad is told to have a high structural integrity and resistance to lamination. The thermoplastic film also provides a fluid barrier function.

Through WO 01/12902 there is known a multi-ply tissue product in which an inner layer of passively bonded, hydrophilic fibers is interposed between two outer cellulosic tissue paper plies. The outer plies may be joined together along their longitudinal edges. This tissue product is mainly intended as a napkin to stop the passage of bodily fluids during sneezing, nose blowing etc. and it is told to provide an improved barrier to prevent these fluids from wetting the hands of the user.

### OBJECT AND MOST IMPORTANT FEATURES OF THE INVENTION

The object of the present invention is to provide a fibrous web product in which a high bulk material is incorporated and kept between plies of fibrous web material. A functional substance, for example a cleaning, polishing, disinfectant, skin treating, medical treatment and/or deodorizing substance, should be integrated in the fibrous web product and be released from the product upon use when a pressure is applied thereto. The product should be adapted to be used in the form of web sections of a defined length, for example as cosmetic pads or high bulk wipes.

According to the invention this object has been solved by the fact that a barrier layer which is at least partly liquid impervious is applied between at least one of said outer plies and the inner material layer and that at least one microencapsulated functional substance is applied between said barrier layer and said at least one outer ply.

Said microencapsulated functional substance is preferably chosen from the following group of substances: cleaning, polishing, disinfectant, skin treating, medical treatment, deodorizing substances.

Further features of the invention are disclosed in the following description and in the claims.

### DESCRIPTION OF DRAWINGS

The invention will in the following be closer described with reference to some embodiments shown in the accompanying drawings.
Fig. 1 shows a schematic view through a web section forming a tissue product containing an encapsulated functional substance.
Fig. 2 is a corresponding view but illustrating the product under use when pressure is applied thereto.
Fig. 3 illustrates a rolled tissue product containing a plurality of web sections.
Fig. 4 illustrates a stack of individual web sections according to a further embodiment of the invention.

### DESCRIPTION OF AN EMBODIMENT

In the following description the web material will be referred to as a tissue paper web. It is however realized that the invention also includes nonwoven materials.

A tissue paper is defined as a soft absorbent paper having a basis weight below 65 g/m² and typically between 10 and 50 g/m². Its density is typically below 0.60 g/cm³, preferably below 0.30 g/cm³ and more preferably between 0.08 and 0.20 g/cm³. Moist tissue paper webs are usually dried against one or more heated rolls. A method commonly used for tissue paper is so called yankee drying. At yankee drying the moist paper web is pressed against a steam-heated yankee cylinder, which can have a very large diameter. The paper web is usually creped against the yankee cylinder.

Another drying method is so called through-air-drying (TAD). In this method the paper is dried by means of hot air blown through the moist paper web, often without a preceding wet pressing. In connection with the TAD drying the patterned structure of the drying fabric is transferred to the paper web. This structure is essentially maintained also in wet condition of the paper, since it has been imparted to the wet paper web.

In the international patent application no. PCT/SE98/02461 there is disclosed a method for producing an impulse dried paper, especially tissue paper, having a three-dimensional pattern, said paper having high bulk and softness. Impulse drying shortly involves that the moist paper web is passed through the press nip between a press roll or press shoe and a heated roll, which is heated to such a high temperature that a quick and strong steam generation occurs in the interface between the moist paper web and the heated roll. The three-dimensional embossment pattern is accomplished by means of a pattern provided on the heated roll. The counter means, for example a press felt, against which the paper is pressed in connection with the simultaneous impulse drying and shaping, has a non-rigid surface.

The present invention refers to all types of tissue paper. The tissue paper may be creped or non-creped. The creping may take place in wet or dry condition. It may further be foreshortened by any other methods, such as so called rush transfer between wires.

The fibers contained in the tissue paper are mainly pulp fibers from chemical pulp, mechanical pulp, thermo mechanical pulp, chemo mechanical pulp and/or chemo thermo mechanical pulp (CTMP). The fibers may also be recycled fibers. The tissue paper may also contain other types of fibers enhancing e.g. strength, absorption or softness of the paper. These fibers may be made from regenerated cellulose or synthetic material such as polyolefins, polyesters, polyamides etc.

The tissue paper coming out from the tissue machine as a single-ply paper sheet may be converted to the final tissue product in many ways, for example embossed, laminated to a multi-ply product, rolled or folded. A laminated multi-ply tissue product comprises at least two tissue plies, which are often joined by either adhesive or mechanically. The adhesive may be applied all over the paper or just in regions, for example dots or lines, or only along the edges of the product. The mechanical methods are mainly embossing either over the entire area of the plies or only along the edges, so called edge embossing. In the final product the different plies as mostly easy detectable and may often be separated from each other as single plies.

The tissue paper coming out from the tissue paper machine may further comprise one or more layers. In the case of more than one layer this is accomplished either in a multi-layered headbox, by forming a new layer on top of an already formed layer or by couching together already formed layers. These layers cannot or only with considerable difficulty be separated from each other and are joined mainly by hydrogen bonds. The different layers may be identical or may have different properties regarding for example fibre composition and chemical composition.

The term nonwoven is applied to a wide range of products, which in term of their properties are located between the groups of paper and cardboard on the one hand and textiles on the other hand. As regards nonwoven a large number of extremely varied production processes are used, such as the air-laid, wetlaid, spunlaced, spunbond, meltblown techniques etc. Nonwoven materials represent flexible porous fabrics that are not produced by the classical methods of weaving or knitting, but by intertwining and/or by cohesive and/or adhesive bonding of typical synthetic textile fibers, which may for example be present in the form of endless fibers or fibers prefabricated with an endless length, as synthetic fibers produced in situ or In the form of staple fibers. Alternatively they may be made from natural fibers or from blends of synthetic fibers and natural fibers.

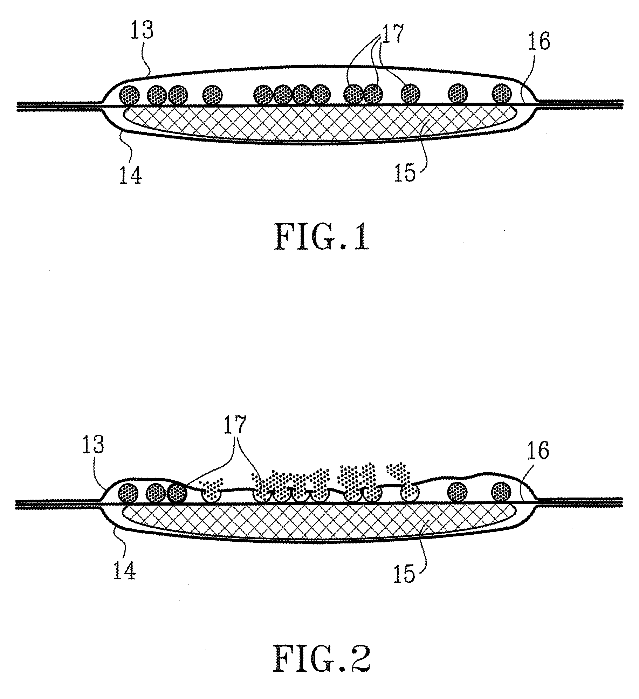

The tissue product 12 according to the invention and as shown in Fig. 1 comprises at least two outer plies 13 and 14 of tissue or nonwoven and an inner material layer 15 interposed between the outer plies. The inner material layer 15 comprises a bulky soft material and is disposed in discrete spaced apart areas between the outer tissue plies. A barrier layer 16 is applied between one outer ply 13 and the inner material layer 15. Microcapsules 17 of a functional substance are applied between the barrier layer 16 and said outer ply 13. The functional substance can be any optional substance having a desired function, such as cleaning, polishing, disinfectant, skin treating, cosmetic, medical treatment and/or deodorizing function.

Microencapsulation of chemical substances is well-known in many technical fields and involves that the substance is encapsulated with a polymeric material which is pressure sensitive. This means that the microcapsules are ruptured when exerted to a certain pressure, which will cause the substance to be released.

The barrier layer 16 is of a material that is at least substantially liquid impervious, such as a polymeric film, a hydrophobic tissue or nonwoven material. Preferably the barrier layer 16 is of a biologically degradable material. The barrier layer 16 should prevent the functional substance from being absorbed into the bulky inner material layer 15. It is herewith also prevented that the functional substance penetrates through the opposite ply 14 and smears the user's fingers.

The inner bulky soft material may be of different kind. One preferred example is a hydrophilic low density fibrous material. These can include natural fibers such as cellulosic fluff pulp and cotton, regenerated fibers such as viscose or synthetic fibers of different kind. The fibers may be unbonded, which means that they are not actively bonded by any bonding technique. They may alternatively be bonded by a bonding technique maintaining a bulky structure of the fibrous material, for example through-air-bonding. A bulky, low density fibrous material in this respect is defined as a material having a dry density of no more than 0,1 g/cm³, preferably no more than 0,08 g/cm³. The term "dry" in this respect means a moisture content of 10% by weight and lower.

In another embodiment the bulky, low density material is a foam material.

In a still further embodiment the bulky, low density material comprises at least one ply of cellulosic web material 18 having a three-dimensional structure, such as embossing pattern, corrugations or the like. The cellulosic web material may be a tissue paper. Preferably two or more tissue plies are provided to form said inner bulky layer. It is preferred that the three-dimensional structure of the inner tissue plies is substantially wet stable. This may be provided by imparting the three-dimensional shape simultaneously with drying a wet web, for example by through-air-drying or impulse drying a paper web while this is in contact with a pattern wire, belt or roll.

The basis weight of a single ply of a tissue product according to the invention may vary from 13 to 30 g/cm². The tissue product may of course comprise more than one ply.

The ply 13 is liquid permeable and lets the functional substance through when this has been released from the microcapsules.

When the product is used and pressure is applied to the ply 13, the microcapsules 17 are ruptured and the functional substance is released, passes through the ply 13 and is available on the outside of the ply 13, as is illustrated in Fig. 2.

The product may be used either in dry or wet condition.

If for any reason it is desired to have a functional substance available from both sides of the product, an additional barrier layer is applied on the opposite side of the bulky inner material layer 15 and additional microencapsulated functional substance, which may either be the same or different from the first functional substance, is applied between the additional barrier layer and the ply 14.

In one embodiment of the invention an added absorbent material may be applied between the plies of the tissue product, for example in the manner disclosed in WO 02/42559. This added absorbent material may be selected from the following materials: absorbent clay, e.g. bentonite, superabsorbent hydrogel materials, natural cellulose fiber materias, e.g. chemical pulp, chemomechanical pulp, chemothermomechanical pulp (CTMP) and DFR (dry formed reel pulp).

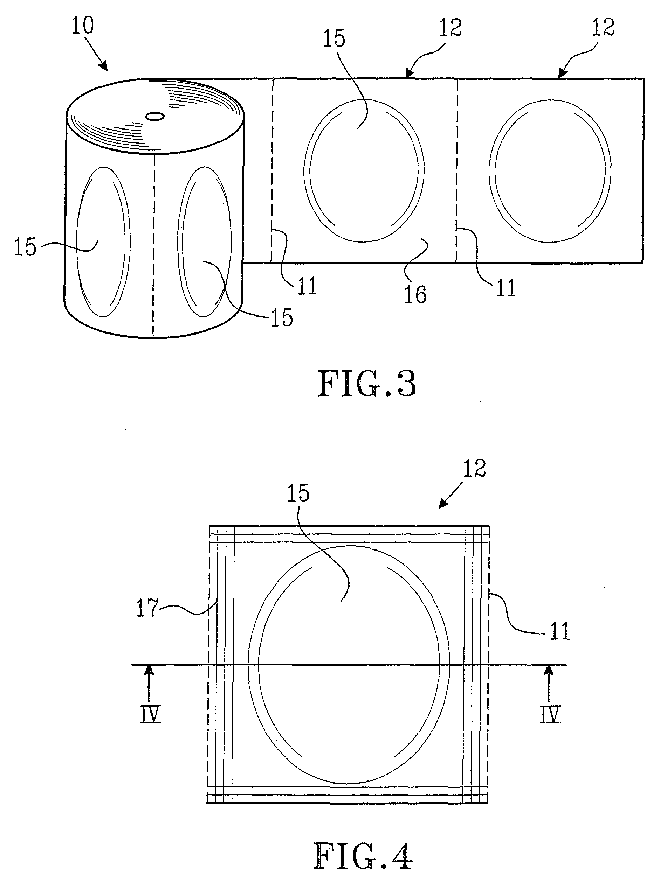

With reference to Fig. 3 the tissue products 12 are shown as a rolled product 10, which is perforated along perforation lines 11 to define a plurality of individual web sections or tissue products 12, where each web section is removable for use. With reference to Fig. 4 the invention is illustrated as a stack of individual web sections or tissue products 12 which have been cut from a web length of material. In an alternative embodiment the individual web sections may be folded and optionally interleaved into a stack suitable for dispensing from a container in a manner, which is known per se.

The perforations 11 or cuts in the outer plies 13 and 14 defining the individual web sections are preferably provided in areas, which are substantially free from said inner bulky soft material 15. The tissue plies 13 and 14 preferably have a larger surface area in the plane than the inner bulky soft material 15 and extend outside the edges thereof. The tissue plies 13 and 14 are joined in these extending portions 18 for example by embossing, gluing or a combination of both. Such edge embossing and/or glue lines are indicated with the numeral 19 in Fig. 3. The inner bulky soft material 15 thus preferably forms discrete pads disposed in the central portion of the respective web section 12, said pads 15 being surrounded by a rim 18 of the tissue plies 13 and 14 which in at least portions of said rim 18 are joined to each other. It is also understood that each web section 12 may comprise two or more pads 15 of bulky soft material.

The process for making the tissue product involves bringing the pads 15 in a registered system between the tissue plies 13 and 14 and subsequently joining the plies by embossing and/or gluing in the areas 18. Perforations or cuts are further provided to define the desired web sections.

In one aspect of the invention the pads 15 of bulky soft material should take up an area of less than 90%, preferably less than 80% and more preferably less than 75%, of the total area of a respective web section 12, so as to leave a rim 18 of sufficient size for embossing and/or gluing.

In an alternative embodiment the bulky soft material 15 is applied as a continuous web material between the tissue plies 13 and 14, wherein the perforations or cuts defining the individual web sections 12 extend through also said inner material layer 15.

Joining of the plies 13 and 14 may be accomplished by other methods as well, such as thermobonding, ultrasonic or laser welding, in case thermoplastic fibers are comprised in the plies. As stated above the plies can be of other fibrous materials than tissue paper, such as any kind of nonwoven materials.

It is understood that the sizes of the tissue products, web sections 12, may vary depending on the intended use. For a cosmetic pad a standard size of about 80 x 70 mm suggested, while for a wipe a standard size of about 260 x 180 mm may be used. The above formats are however only examples and the size may be optionally chosen.

## Claims

1. A fibrous web product comprising at least two outer plies (13,14) of fibrous web material and an inner material layer (15) interposed between the outer plies, said inner material layer comprising a bulky soft material,
**characterized in**
**that** a barrier layer (16) which is at least substantially liquid impervious is applied between at least one of said outer plies (13,14) and the inner material layer (15) and that at least one microencapsulated functional substance (17) is applied between said barrier layer (16) and said at least one outer ply (13).

2. A fibrous web product as claimed in claim 1,
**characterized in**
**that** said microencapsulated functional substance (17) is chosen from the following group of substances: cleaning, polishing, disinfectant, skin treating, cosmetic, medical treatment, deodorizing substances.

3. A fibrous web product as claimed in claim 1 or 2,
**characterized in**
**that** said inner material layer (15) is disposed in discrete spaced apart areas between said outer fibrous web material plies (13,14) and that said outer plies are provided with perforations (11) or alternatively are cut between said spaced apart areas containing said inner material layer (15), said perforations or cuts defining individual web sections (12) and are provided in areas which are substantially free from said inner material layer (15).

4. A fibrous web product as claimed in claim 3,
**characterized in**
**that** said inner material layer (15) is disposed in the form of at least one discrete pad in each of said individual web sections (12).

5. A fibrous web product as claimed in any of the preceding claims,
**characterized in**
**that** said inner material layer (15) comprises a hydrophilic low density fibrous or foam material.

6. A fibrous web product as claimed in any of claims 1-5,
**characterized in**
**that** said inner material layer (15) comprises at least one ply of cellulosic web material (18) having a three-dimensional structure, such as embossing pattern, corrugations or the like.

7. A fibrous web product as claimed in claim 6,
**characterized in**
**that** said three-dimensional structure is substantially wet stable.

8. A fibrous web product as claimed in any of the preceding claims,
**characterized in**
**that** the outer plies (13,14) of fibrous web material are joined together along at least a substantial part of the periphery of each individual web section (12) by embossing, adhesive or the like.

9. A fibrous web product as claimed in claim 8,
**characterized in**
**that** the outer plies (13,14) of fibrous web material are joined together along the entire periphery of each individual web section (12) by embossing, adhesive (17) or the like so as to enclose the at least one pad (15) by a rim (16) of said outer plies (13, 14) being joined together.

10. A fibrous web product as claimed in any of the preceding claims,
**characterized in**
it is a roll product (10) comprising a plurality of perforation lines (11) defining individual web sections (12) each holding at least one pad (15) of inner material layer.

11. A fibrous web product as claimed in any of claims 1-9,
**characterized in**
**that** the product is a stack of web sections (12) which optionally are folded.
